Europäisches Patentamt

European Patent Office

Office européen des brevets

(11) Numéro de publication: **0 493 220 B1**

## (12) FASCICULE DE BREVET EUROPEEN

(45) Date de publication du fascicule du brevet: **11.10.95**    (51) Int. Cl.⁶: **A61N 1/365**

(21) Numéro de dépôt: **91403481.4**

(22) Date de dépôt: **20.12.91**

(54) **Procédé de contrôle d'un stimulateur cardiaque.**

(30) Priorité: **27.12.90 FR 9016292**

(43) Date de publication de la demande:
**01.07.92 Bulletin 92/27**

(45) Mention de la délivrance du brevet:
**11.10.95 Bulletin 95/41**

(84) Etats contractants désignés:
**BE CH DE ES GB IT LI**

(56) Documents cités:
**EP-A- 0 178 528**
**US-A- 4 702 253**
**US-A- 4 730 619**

(73) Titulaire: **ELA MEDICAL (Société anonyme)**
**98, Rue Maurice Arnoux**
**F-92541 Montrouge (FR)**

(72) Inventeur: **Bonnet, Jean-Luc**
**3, Avenue Richerand**
**F-75010 Paris (FR)**

(74) Mandataire: **Laget, Jean-Loup**
**Cabinet Pierre Loyer**
**77, rue Boissière**
**F-75116 Paris (FR)**

## Description

L'invention concerne un procédé de contrôle d'un stimulateur cardiaque et plus particulièrement d'un stimulateur cardiaque à fréquence de stimulation asservie à un indicateur de la demande métabolique, c'est-à-dire au niveau d'activité du patient porteur du stimulateur.

Lors d'un effort, certains paramètres physiologiques sont modifiés : ce sont des indicateurs de l'effort, qui donnent une information sur la demande métabolique. Cette information est traitée au moyen d'un algorithme qui en déduit une fréquence de stimulation cardiaque, grâce à une relation entre le paramètre et la fréquence cardiaque.

Dans la plupart des stimulateurs cardiaques, on effectue périodiquement une mesure d'un paramètre et on en déduit une augmentation, une baisse, ou un maintien, de la fréquence cardiaque. Il peut arriver que le paramètre ait une variabilité physiologique non négligeable d'une mesure à la suivante, et induise une erreur d'interprétation sur l'activité réelle du patient.

Par exemple, si le paramètre est la ventilation-minute, c'est-à-dire le volume d'air expiré en une minute, il présente, en période d'effort, des irrégularités appelées "trous ventilatoires" correspondant à des changements de rythme de la respiration, qui induisent une baisse de la ventilation-minute pendant quelques cycles respiratoires.

La conséquence directe dans un système asservi, est une baisse momentanée de la fréquence cardiaque, alors que le patient est encore en activité, ce qui est préjudiciable.

Un but de la présente invention est de proposer un contrôle de la variation du paramètre avant d'agir sur la fréquence cardiaque, de façon à éviter les variations prématurées de la fréquence de stimulation.

Un autre but de la présente invention est de proposer un contrôle de la variation du paramètre en distinguant le cas de l'augmentation de la valeur du paramètre susceptible d'entraîner une augmentation de la fréquence cardiaque, et le cas de la diminution de la valeur du paramètre, susceptible d'entraîner une diminution de la fréquence cardiaque.

La présente invention a pour objet un procédé de contrôle d'un stimulateur cardiaque à fréquence de stimulation asservie à la demande métabolique, dans lequel on calcule la fréquence cardiaque FC, tous les quatre cycles cardiaques, au moyen d'une relation linéaire avec la ventilation-minute, caractérisé en ce que :

- on mesure la ventilation-minute moyenne VE-8-0 sur les huit derniers cycles respiratoires (1er à 8e),

- on la compare à la ventilation-minute moyenne VE-8-1 sur les huit cycles respiratoires antérieurs (9e à 16e),
- et on détermine que l'on est en phase d'effort lorsque VE-8-0 > VE-8-1, et en phase de récupération lorsque VE-8-0 < VE-8-1.

Selon d'autres caractéristiques de l'invention :
- en phase d'effort, on maintient la fréquence de stimulation cardiaque à sa valeur lorsqu'un trou ventilatoire correspondant à une baisse temporaire de la ventilation-minute entraîne le calcul d'une fréquence cardiaque d'asservissement en diminution ;
- on maintient la fréquence de stimulation cardiaque à sa valeur, en cas de diminution de la fréquence cardiaque d'asservissement calculée, jusqu'à la confirmation du passage à l'état de récupération par la comparaison de VE-8-0 et VE-8-1 ;
- en phase de récupération, la fréquence de stimulation cardiaque est réduite en respectant une pente de décélération ;
- en phase de récupération, on garde en mémoire la fréquence cardiaque d'asservissement pendant quatre cycles respiratoires, pour rester en phase de récupération en cas d'artefact de ventilation inférieur à quatre cycles respiratoires ;

D'autres caractéristiques ressortent de la description qui suit faite avec référence aux dessins annexés sur lesquels on peut voir :

Fig 1 une courbe représentative de la ventilation-minute d'un patient durant un effort ;

Fig 2 un diagramme représentatif de la fréquence de stimulation cardiaque en réponse aux variations de la ventilation-minute de la Fig. 1 ;

Fig 3 un diagramme représentatif de la ventilation-minute en variation croissante ;

Fig 4 un diagramme représentatif de la ventilation-minute en variation décroissante ;

Fig 5 un diagramme représentatif de la ventilation-minute en variation croissante avec apparition d'un trou ventilatoire ;

Fig 6 un diagramme représentatif des variations de la fréquence de stimulation cardiaque en cours d'effort, en présence d'un trou ventilatoire;

Fig 7 un diagramme représentatif des variations de la fréquence cardiaque en fin d'effort ;

Fig 8 un diagramme représentatif des variations de la fréquence cardiaque en cas de rebond de la ventilation en phase de récupération ;

Fig 9 un diagramme représentatif des variations de la fréquence cardiaque en cas de reprise d'effort en phase de récupération ;

Fig 10 un diagramme d'états du stimulateur cardiaque.

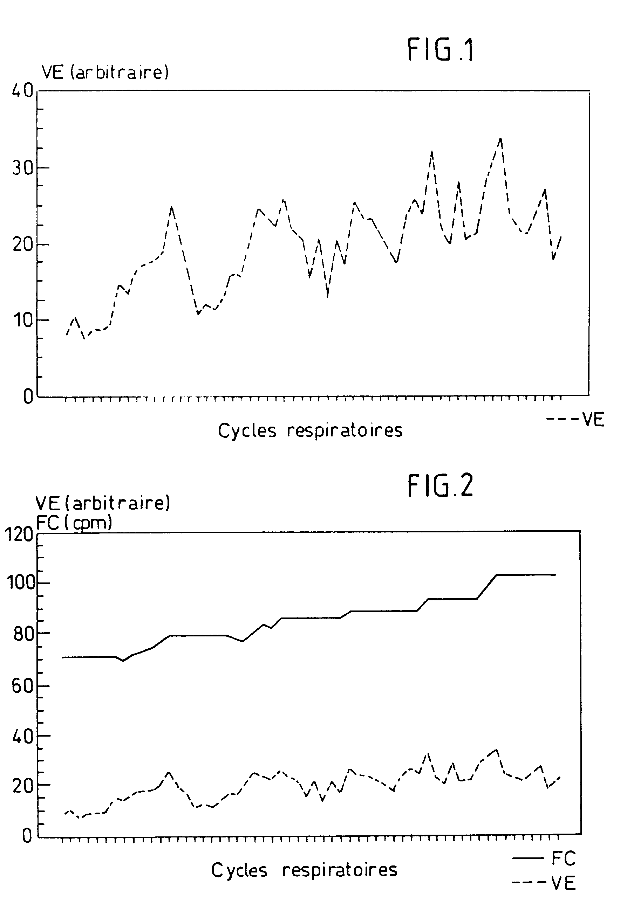

La fig 1 montre la ventilation-minute VE enregistrée par un capteur, durant un test d'effort. VE est calculée à chaque cycle respiratoire, sur la moyenne des quatre cycles précédents. Le diagramme montre de façon très nette des trous ventilatoires.

La fig 2 montre sur la courbe FC une simulation d'asservissement de la fréquence cardiaque FC à la ventilation-minute VE. Malgré la présence des trous ventilatoires durant l'effort, la réponse en fréquence est relativement progressive. Mais des trous de fréquence cardiaque apparaissent durant la phase de montée du niveau d'activité, alors que, physiologiquement, la fréquence cardiaque a une très faible variabilité à l'effort.

L'un des buts de l'invention est d'éliminer la survenue d'une baisse de fréquence cardiaque durant une phase d'effort. Le problème est de différencier un trou ventilatoire d'une réelle fin d'effort.

A cet effet, on analyse la ventilation-minute VE et ses valeurs moyennes sur un certain nombre de cycles respiratoires :

VE-4 : moyenne des 4 dernières VE mesurées ;

VE-8-0 : moyenne des 8 dernières VE mesurées ;

VE-8-1 : moyenne des 8 VE mesurées entre les 9e et 16e cycles respiratoires précédents.

Le principe de l'algorithme de discrimination est basé sur l'évolution des valeurs de VE-8-0 et VE-8-1.

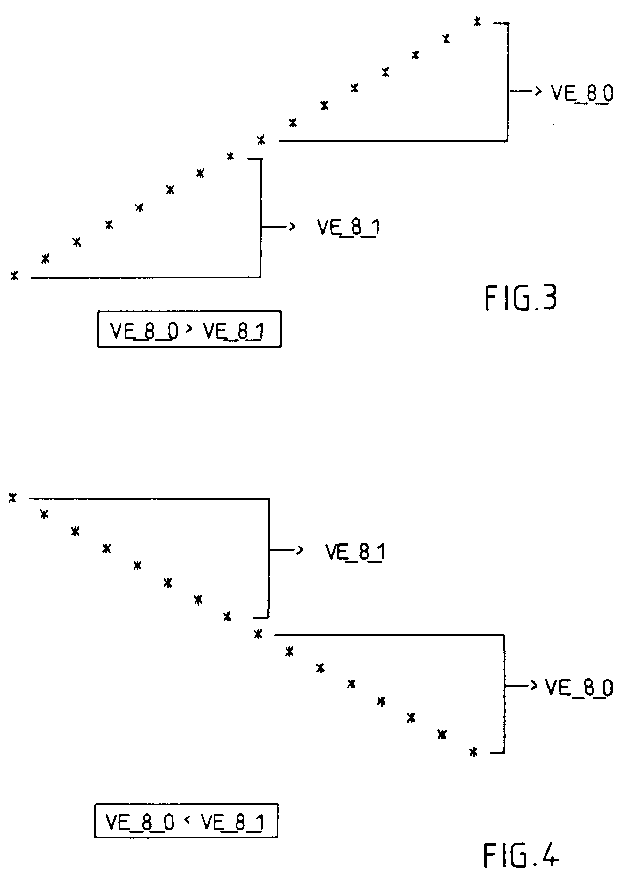

Sur la Fig. 3, la ventilation est croissante et VE-8-0 est supérieure à VE-8-1.

Sur la Fig. 4, la ventilation est décroissante et VE-8-0 est inférieure à VE-8-1.

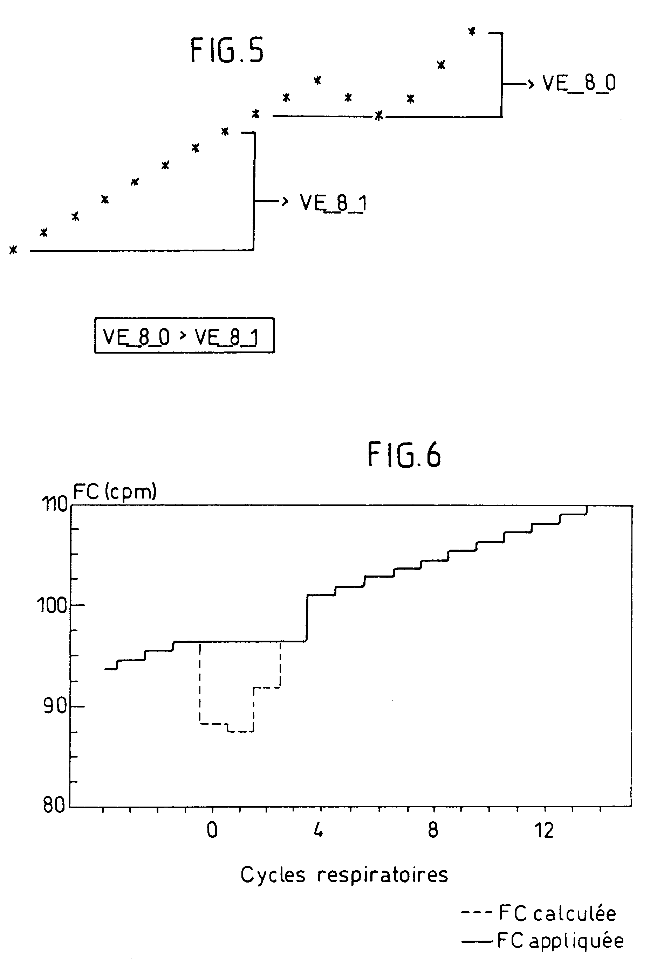

Sur la Fig. 5, la ventilation est généralement croissante et on est en présence d'un trou ventilatoire de quelques cycles : on constate que VE-8-0 reste supérieure à VE-8-1.

Par la comparaison de la ventilation-minute moyenne sur 8 cycles et de la ventilation-minute moyenne sur les 8 cycles antérieurs, on peut ainsi distinguer une véritable fin d'effort (VE-8-0 < VE-8-1) d'un trou ventilatoire (VE-8-0 > VE-8-1).

La figure 6 représente les variations de la fréquence cardiaque en cours d'effort, calculée par la relation d'asservissement à la ventilation-minute mesurée par le capteur. La fréquence cardiaque calculée suit la variation de la ventilation-minute, et en cas de trou ventilatoire, cette fréquence diminue selon la ligne en pointillés. Selon l'invention, la fréquence cardiaque est maintenue à sa dernière valeur et elle reprend sa progression dès que l'activité ventilatoire reprend. De cette façon, est évitée une baisse temporaire de la fréquence cardiaque en phase d'effort.

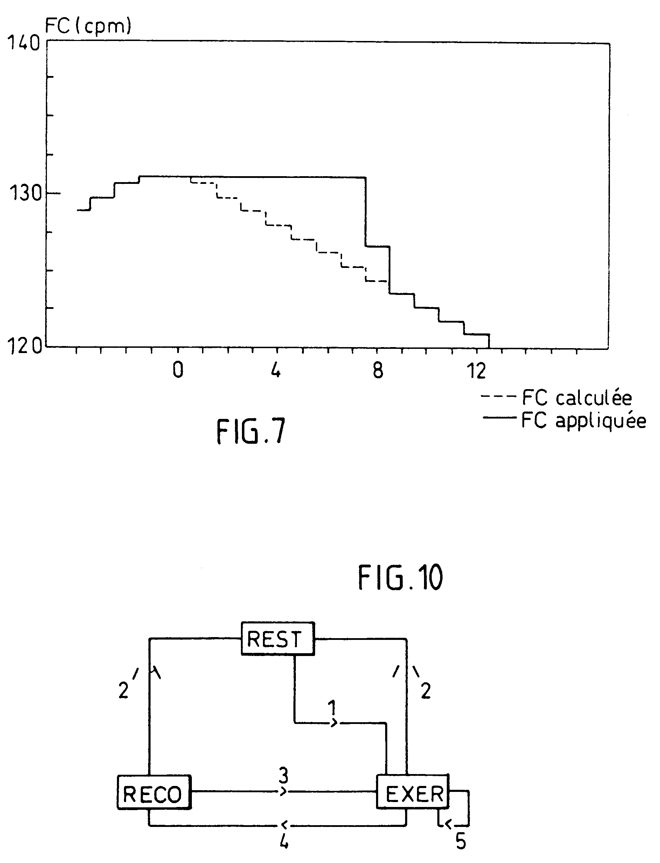

La figure 7 représente les variations de la fréquence cardiaque en fin d'effort, la ligne en pointillés représentant la fréquence cardiaque calculée par la relation d'asservissement. Lorsque, en fin d'effort, la ventilation-minute diminue, la fréquence cardiaque diminue également. Selon l'invention, la fréquence cardiaque est maintenue à sa dernière valeur jusqu'à confirmation de la fin d'effort.

La phase dite de récupération est située entre la fin de l'effort et le retour à l'état de repos.

Pendant cette phase, la ventilation-minute diminue afin de retrouver sa valeur de base.

Pour éviter qu'un trou ventilatoire soit perçu comme une fin d'effort et soit immédiatement suivi d'une baisse de la fréquence cardiaque, l'invention met en oeuvre un algorithme de confirmation de la fin d'effort ; jusqu'à cette confirmation, la fréquence cardiaque reste constante, ce qui est représenté par le plateau en trait plein sur la figure 7.

La confirmation de la fin d'effort est obtenue lorsque VE-8-0 devient inférieure à VE-8-1.

Dès que le système à identifié une fin d'effort, il passe dans une phase dite de "récupération". Durant celle-ci, le comportement est différent de celui de l'effort. En effet, la fréquence cardiaque d'asservissement va évoluer en fonction de l'information du capteur de ventilation.

Il faut préciser l'information suivante : que le système soit en phase d'effort ou en phase de récupération, la fréquence cardiaque d'asservissement est limitée par des pentes. On définit une pente d'accélération et une pente de décélération. Celles-ci sont appliquées à chaque recalcul de la fréquence cardiaque d'asservissement, c'est-à-dire, tous les 4 cycles cardiaques. Les pentes sont appliquées sur la période du cycle cardiaque, et sont donc exprimées en millisecondes (ms).

Durant la récupération, la fréquence cardiaque d'asservissement est limitée dans sa décroissance par la pente de décélération. Ainsi, la période de stimulation ne peut pas augmenter plus qu'une valeur programmable (de 16 ou 31 ms tous les 4 cycles cardiaques).

Durant la phase de récupération, l'information du capteur de ventilation n'est pas toujours décroissante. On peut observer une augmentation de la ventilation et donc une augmentation de la fréquence cardiaque. Si celle-ci se prolonge, il est nécessaire de conclure à une reprise de l'effort, et donc de sortir de l'état de récupération pour passer dans l'état d'effort. Par contre, si cette augmentation de ventilation (et donc de fréquence cardiaque d'asservissement) n'est qu'un phénomène transitoire, alors il ne faut pas passer en état d'effort, car dans ce cas, on aurait à nouveau un plateau de fréquence dû à la phase de confirmation de la fin d'effort (Fig. 7).

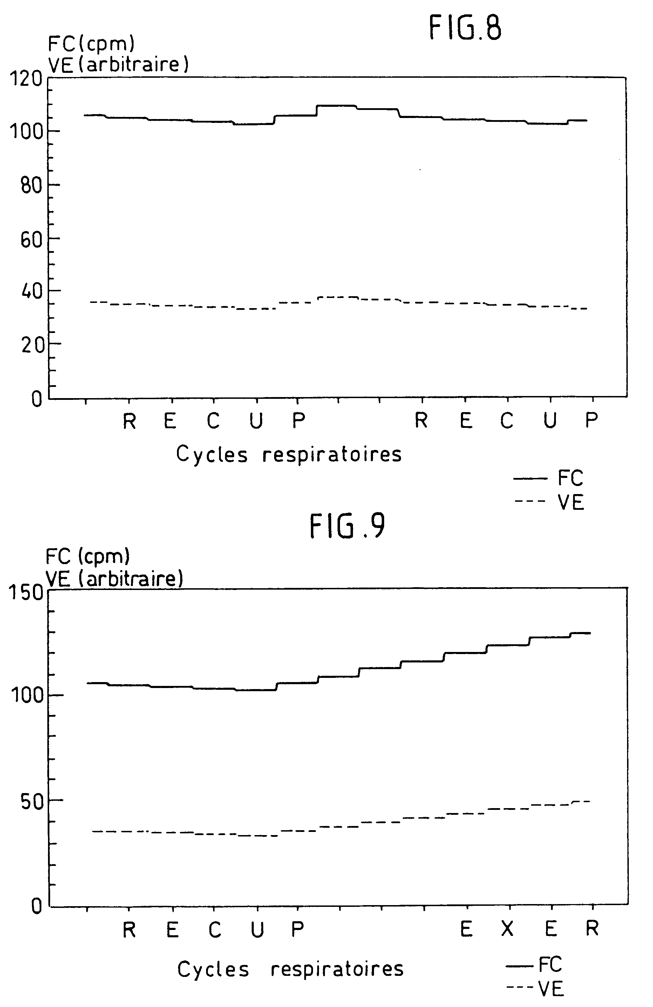

Dès qu'une augmentation de la fréquence cardiaque d'asservissement est observée, on autorise celle-ci. On garde alors en mémoire la plus petite fréquence cardiaque d'asservissement atteinte, dans les cycles précédents. Si la fréquence cardiaque d'asservissement calculée devient inférieure à cette valeur minimale avant le 4ème recalcul de la fréquence cardiaque (celui-ci est effectué tous les quatre cycles cardiaques), alors le système considère qu'il ne s'agit pas d'une reprise d'effort, et reste en récupération (voir fig. 8).

Par contre, si au 4ème recalcul, la fréquence est toujours supérieure à la fréquence minimale atteinte, alors le système considère qu'il y a une reprise d'effort, et passe à nouveau en état d'effort (voir fig. 9). Cela signifie que pour repasser à nouveau en phase de récupération, les conditions décrites précédemment devront être remplies à nouveau, et on observera un plateau de fréquence avant la récupération.

Le procédé de contrôle du stimulateur cardiaque selon l'invention est mis en oeuvre de la façon suivante :
Deux fonctions sont réalisées de façon asynchrone. D'une part, tous les 4 cycles cardiaques une nouvelle fréquence cardiaque d'asservissement est calculée, d'après l'information ventilatoire. D'autre part, tous les 4 cycles respiratoires une nouvelle valeur moyenne de ventilation est calculée (VE-4). Celle-ci sera utilisée pour le calcul de VE-8-0 et VE-8-1.

1) Calcul de la fréquence cardiaque d'asservissement.

La fréquence cardiaque d'asservissement est calculée d'après une relation linéaire établie entre la ventilation-minute et la fréquence cardiaque. Une nouvelle valeur de fréquence de stimulation cardiaque d'asservissement est calculée tous les 4 cycles cardiaques.

Le calcul est effectué à partir de la valeur moyenne de la ventilation sur les 4 dernières valeurs mesurées (VE-4) :

$$FC = a \times VE\text{-}4 + b$$

2/ Etat d'effort

A partir de cette valeur, le système détermine l'état d'effort du patient. On définit trois états : repos (REST), effort (EXER) et récupération (RECO) :
- REST correspond à une fréquence cardiaque calculée sous un certain seuil (période de base + 6%) ;
- EXER correspond à une fréquence cardiaque croissante au dessus du seuil ;
- RECO correspond à une fréquence au dessus du seuil mais décroissante.

Les passages d'un état à l'autre sont dépendants du résultat de certains tests sur l'évolution de la fréquence cardiaque d'asservissement. Les conditions de changement d'états sont schématisées sur le diagramme d'états de la Fig. 10.

Sur ce diagramme, les voies 1 à 5 ont la signification suivante :
- Voie 1 : la fréquence d'asservissement est supérieure au seuil ;
- Voie 2 : la fréquence d'asservissement est inférieure au seuil ;
- Voie 3 : reprise de l'effort (Fig. 9) ;
- Voie 4 : fin d'effort (Fig. 7) ;
- Voie 5 : le critère de fin d'effort n'est pas rempli.

La voie 2, de l'état RECO à l'état REST, est la voie normale de retour au repos à l'issue d'une phase de récupération.

La voie 2, de l'état EXER à l'état REST, correspond au retour rapide à l'état de repos en cas d'artefact de ventilation pendant la phase de récupération (cas de la Fig. 8).

**Revendications**

1. Procédé de contrôle d'un stimulateur cardiaque à fréquence de stimulation asservie à la demande métabolique, dans lequel on calcule la fréquence cardiaque FC, tous les quatre cycles cardiaques, au moyen d'une relation linéaire avec la ventilation-minute, caractérisé en ce que :
   - on mesure la ventilation-minute moyenne VE-8-0 sur les huit derniers cycles respiratoires (1er à 8e),
   - on la compare à la ventiation-minute moyenne VE-8-1 sur les huit cycles respiratoires antérieurs (9e à 16e),
   - et on détermine que l'on est en phase d'effort lorsque VE-8-0 > VE-8-1, et en phase de récupération lorsque VE-8-0 < VE-8-1.

2. Procédé selon la revendication 1, caractérisé en ce qu'en phase d'effort, on maintient la fréquence de stimulation cardiaque à sa valeur lorsqu'un trou ventilatoire correspondant à une baisse temporaire de la ventilation-minute entraîne le calcul d'une fréquence cardiaque d'asservissement en diminution.

3. Procédé selon la revendication 1, caractérisé en ce qu'on maintient la fréquence de stimulation cardiaque à sa valeur, en cas de diminution de la fréquence cardiaque d'asservissement calculée, jusqu'à la confirmation du passage à l'état de récupération par la comparaison de VE-8-0 et VE-8-1.

4. Procédé selon la revendication 3, caractérisé en ce qu'en phase de récupération, la fréquence de stimulation cardiaque est réduite en respectant une pente de décélération.

5. Procédé selon la revendication 4, caractérisé en ce qu'en phase de récupération, on garde en mémoire la fréquence cardiaque d'asservissement pendant quatre cycles respiratoires, pour rester en phase de récupération en cas d'artefact de ventilation inférieur à quatre cycles respiratoires.

## Claims

1. A method for monitoring a cardiac pacemaker with stimulation rate controlled by the metabolic demand, in which the heart rate FC is calculated every four cardiac cycles, by means of a linear relation with the minute ventilation, characterised in that:
   - the average minute ventilation VE-8-0 is measured over the last eight respiratory cycles (1st to 8th),
   - it is compared with the average minute ventilation VE-8-1 over the previous eight respiratory cycles (9th to 16th),
   - and it is determined that one is in the exertion phase when VE-8-0 > VE-8-1, and in the recovery phase when VE-8-0 < VE-8-1.

2. A method according to Claim 1, characterised in that in the exertion phase the rate of cardiac stimulation is kept at its value when a ventilation gap corresponding to a temporary drop in the minute ventilation brings about the calculation of a diminishing controlled heart rate.

3. A method according to Claim 1, characterised in that the cardiac stimulation rate is kept at its value, in the event of diminishing of the controlled heart rate calculated, until the passage to the recovery state is confirmed by the comparison of VE-8-0 and VE-8-1.

4. A method according to Claim 3, characterised in that in the recovery phase the cardiac stimulation rate is reduced while keeping to a deceleration slope.

5. A method according to Claim 4, characterised in that in the recovery phase the controlled heart rate is stored for four respiratory cycles in order to remain in the recovery phase in the event of a ventilation artefact of less than four respiratory cycles.

## Patentansprüche

1. Verfahren zur Steuerung eine Herzschrittmachers mit stoffwechselabhängig gesteuerter Stimulationsfrequenz, bei dem eine Herzfrequenz FC nach jeweils vier Herzzyklen mittels einer linearen Beziehung mit der Minuten-Ventilation berechnet wird,
   **dadurch gekennzeichnet, daß**
   - die mittlere Minuten-Ventilation VE-8-0 über den letzten acht Atmungszyklen (1-ter bis 8-ter) berechnet wird,
   - diese mit der mittleren Minuten-Ventilation VE-8-1 über den vorhergehenden acht Atmungszyklen (9-ter bis 16-ter) verglichen wird,
   - und bestimmt wird, daß eine Belastungsphase vorliegt, wenn VE-8-0 > VE-8-1 ist, und daß eine Erholungsphase vorliegt, wenn VE-8-0 < VE-8-1 ist.

2. Verfahren gemäß Patentanspruch 1,
   **dadurch gekennzeichnet, daß**
   während einer Belastungsphase die Herzstimulationsfrequenz auf ihrem Wert beibehalten wird, wenn ein einem temporären Abfallen der Minuten-Ventilation entsprechendes Ventilations-Loch die Berechnung einer abnehmenden Herzregelungsfrequenz bedingt.

3. Verfahren gemäß Patentanspruch 1,
   **dadurch gekennzeichnet, daß**
   im Fall der Verringerung der berechneten Herzregelungsfrequenz die Herstimulationsfrequenz bis zur Bestätigung des Übergangs zum Erholungszustand aufgrund des Vergleichs von VE-8-0 und VE-8-1 auf ihrem Wert beibehalten wird.

4. Verfahren gemäß Patentanspruch 3,
   **dadurch gekennzeichnet, daß**
   die Herzstimulationsfrequenz während der Erholungsphase unter Beachtung einer Verzögerungsrate verringert wird.

5. Verfahren gemäß Patentanspruch 4,
   **dadurch gekennzeichnet, daß**
   in der Erholungsphase die Herzregelungsfrequenz während vier Atmungszyklen gespeichert wird, um im Fall eines weniger als vier Atmungszyklen umfassenden Ventilations-Artefakts in der Erholungsphase zu verbleiben.

## FIG.1

VE (arbitraire)

Cycles respiratoires

- - - VE

## FIG.2

VE (arbitraire)
FC (cpm)

Cycles respiratoires

——— FC

- - - VE

VE_8_0

VE_8_1

VE_8_0 > VE_8_1

FIG.3

VE_8_1

VE_8_0

VE_8_0 < VE_8_1

FIG.4

# FIG.5

VE_8_0

VE_8_1

VE_8_0 > VE_8_1

# FIG.6

FC (cpm)

Cycles respiratoires

---FC calculée
——FC appliquée

FC (cpm)

FIG.7

- - - FC calculée
—— FC appliquée

FIG.10

REST

RECO

EXER

## FIG.8

FC(cpm)
VE(arbitraire)

Cycles respiratoires

—— FC
--- VE

## FIG.9

FC (cpm)
VE (arbitraire)

Cycles respiratoires

—— FC
--- VE